# EUROPEAN PATENT APPLICATION

(11) **EP 2 158 916 A1**
(43) Date of publication of application: **03.03.2010**
(21) Application number: 08163117.8
(22) Date of filing: 27.08.2008
(51) Int. Cl.: A61K 35/74, A61P 15/02, A61P 31/10

(54) **Administration unit comprising lactic acid bacteria**

(71) Applicant: Ellen Aktiebolag, 182 33 Danderyd (SE)
(72) Inventor: Daroczy, Katalin, 632 21 ESKILSTUNA (SE); Weiner Jiffer, Anna, 182 33 DANDERYD (SE)
(74) Representative: Andersson, Inga-Lill

(57) **Abstract**

The present invention relates to an administration unit comprising a core comprising at least one lactic acid bacteria. The core is surrounded by a first envelope. The inventive administration unit is suitable for use in the prophylaxis and/or treatment of a disorder in the urogenital tract.

## Description

### Field of the invention

The present invention relates to an administration unit comprising a core comprising at least one lactic acid bacteria. The core is surrounded by a first envelope.

### Background of the invention

The skin of the urogenital tract and the urogenital mucus membranes of a healthy woman host a specific flora of beneficial and/or commensal microorganisms, such as various species of Lactobacillus. However, the urogenital tract can also be colonised by disease-causing microorganisms. The colonisation of such undesired microorganisms may be a result of sexual transmission, it may occur spontaneously or it may be the result of a disturbed normal microbial flora. The latter is, for instance, known to happen after certain antibiotic therapies.

Furthermore, the vaginal bacterial flora is affected by menstruation and sexual intercourse, and the addition of blood and sperm increase the pH in the vagina. These fluids contain a lot of proteins, which may be digested by harmful bacteria (e.g. Gardnerella vagnalis and Mobiluncus), which establish in the vagina under conditions of increased pH. Degradation products, such as amines (e.g. putrescine and cadacerine) are then produced. At increased pH, these amines become volatile and present a "fishy" odour. This is typically associated with symptoms such as increased vaginal discharge and irritation. This condition is called bacterial vaginosis (BV)

During BV, a shift in the bacterial flora naturally present in the vagina (Lactobacillus dominated) to a biota in which G. vaginalis and anaerobic bacteria predominate is observed.

Many women experience problems with a disturbed vaginal bacterial flora and the most common vaginal disorders are bacterial vaginosis and Candida infections.

BV is caused by an imbalance of the naturally occurring bacterial flora, whereas Candida infections are caused by various species of the yeast Candida, e.g. Candida albicans.

Many studies have shown that lactic acid producting bacteria have an inhibitory effect on the organisms associated with BV, such as G.vaginalis, E.coli, Mobiluncus and Prevotella bivia. A vaginal flora dominated by LAB is also suggested to be of importance as a defence mechanism against Candida infections. LAB may render the vaginal epithelial cells and make them less susceptible for Candida adhesion. Consumption of antibiotics that effectively kill lactic acid bacteria is associated with Candida overgrowth, which also argues for LAB as protection against Candida.

The protective role of LAB has been attributed to their ability to create an acidic environment, secrete inhibitory substances, such as lactic acid, hydrogen peroxide, bacteriocins, etc, as well as to physically compete for space and substrate in the vagina.

It is known to impregnate absorbent articles, such as tampons and sanitary napkins, with lactic acid producing bacteria for the purpose of preserving a normal flora of microorganisms in the urogenital tract of women, and thereby preventing urogenital infections, or regenerating a normal flora of microorganisms in the urogenital tract of women. Such products are e.g. disclosed in EP 0 594 628 and EP 1 322 346.

Furthermore, prior art describe formulations, such as suspensions, suppositories and gelatine capsules, comprising viable lactic acid producing bacteria. Such formulations are for instance disclosed in US 5 466 463 and WO 9 309 793.

US 2004/253217 discloses novel isolated strains of lactic acid producing bacteria of the genera Lactobacillus and Pediococcus, and a method for isolation of such bacterial strains, having the ability to colonise and become established in a human vagina, even during menstrual discharge. The strains are selected from the group consisting of the strain of Lactobacillus gasseri, LN 40, deposited under number LMG P-20560, the strain of Lactobacillus casei subsp rhamnosus, LN 113, deposited under number LMG P-20562, the strain of Lactobacillus fermentum, LN 99, deposited under number LMG P-20561, the strain of Lactobacillus crispatus, LN 01, deposited under number LMG P-20558, and the strain of Pediococcus acidilactici, LN 23, deposited under number LMG P-20559.

As mentioned hereinbefore, bacterial vaginosis and Candida infections are the two most common vaginal infections. However, the treatment and prevention regimens currently available and used typically do not distinguish between these disorders. Hence, a woman suffering from bacterial vaginosis is often treated the same way as one afflicted with the yeast infection Candida.

Accordingly, there is a need in the art to find a more selective therapy for the treatment and/or prophylaxis of bacterial vaginosis, and Candida infections, respectively.

Furthermore, there is a need to find an alternative therapy comprising the administration of lactic acid producing bacteria (LAB). Such a therapy should be well tolerated, convenient and comfortable to administer and provide an improved preventative and inhibitory effect on undesired pathogens.

### Summary of the invention

One purpose of the present invention is to provide a therapy, wherein the preventative effect of lactic acid producing bacteria on undesired microorganisms present in the vagina, is improved. This therapy should be easy, convenient and comfortable to administer and provide excellent patients' acceptability.

This aim is achieved by an administration unit comprising a core comprising at least one lactic acid producing bacteria. The core is surrounded by a first envelope comprising at least one biodegradable and biocompatible polymer.

An administration unit according to the present invention is highly efficacious in killing undesired pathogens in the vagina and helps to maintain and re-establish a healthy and non-pathogenic bacterial flora in the vagina.

The first envelope comprises at least one biodegradable and biocompatible polymer, which gradually degrades in the vaginal environment. This is primarily due to the effect of moisture. Lactic acid producing bacteria are thereby released to exert a preventative effect against vaginal disorders, such as bacterial vaginosis and Candida infections.

When the lactic acid producing bacteria are released in the urogenital tract they propagate, and the advantages of lactic acid producing bacteria according to the introduction are thus obtained.

The lactic acid producing bacteria create an acidic environment and secrete hydrogen peroxide and antimicriobial substances, eg. bacteriocins, thereby inhibiting undesired pathogens present in the vagina.

An administration unit according to the invention is substantially free of unpleasant side effects, such as burning and itching.

In embodiments of the invention, the at least one biodegradable and biocompatible polymer exerts a pH-buffering effect upon degradation in the vagina. Typically, this pH-buffering effect is effected by lowering the pH of the vagina. A favourable environment for the lactic acid bacteria is thereby created when these are released in the vaginal milieu.

A lower pH makes it easier for the lactic acid producing bacteria to recolonize the vagina and thereby restore the natural resistance toward overgrowth by bacteria connected with e.g. BV.

Preferably, the at least one biodegradable and biocompatible polymer is polylactic acid.

Polylactic acid is a biodegradable and biocompatible polymer, which upon contact with water, i.e. moist in the vaginal environment is hydrolysed into lactic acid. Lactic acid is thereby formed and released in the vagina. Hence, the pH-lowering effect of the biodegradable and biocompatible polymer is attributed to the formation of lactic acid, which not only serves to lower the pH in the vagina, but may also function as a growth factor for lactic acid bacteria. Accordingly, lactic acid creates an ideal environment for the lactic acid bacteria when these are released in the vagina.

Another important advantage of polylactic acid is associated with its ability to adhere well to epithelial cells in the vagina.

In preferred embodiments, the administration unit according to the invention is administered vaginally. This ensures an improved local effect in the vagina, where the protective mechanisms are to be exerted.

Typically, the lactic acid producing bacteria are freeze dried. Freeze drying is a gentle process which helps to preserve the bacterial protein structure. Hence, the bacterial survival is thereby improved.

Typically, the lactic acid producing bacteria are selected from the genus Lactobacillus, Pediococcus, Lactococcus and Leuconostoc.

These genera are known to have a beneficial effect in the vaginal milieu.

Another object of the present invention is to find a more selective therapy for the treatment and/or prophylaxis of bacterial vaginosis, and Candida infections, respectively.

According to one embodiment of the present invention, the lactic acid bacteria is Lactobacillus fermentum.

Preferably, the strain of Lactobacillus fermentum is the strain Lactobacillus fermentum, LN 99, deposited by the Applicant under number LMG P-20561.

The present inventors have found that Lactobacillus fermentum, especially the strain LN 99 is particularly effective for the treatment and/or prophylaxis of Candida infections.

Accordingly, an administration unit comprising L. fermentum, and especially LN 99 may be administered to women afflicted with Candida infections.

In another embodiment of the invention, the lactic acid producing bacteria is a strain of Lactobacillus casei, especially the strain Lactobacillus casei subsp rhamnosus, LN 113, deposited under number LMG P-20562. The present inventors have found that L. casei, and especially LN 113 is particularly effective for the treatment and/or prophylaxis of bacterial vaginosis. Hence, an administration unit comprising L. casei, e.g. LN 113 may be administered to women afflicted by bacterial vaginosis.

As mentioned hereinbefore, women afflicted with bacterial vaginosis are often treated the same way as women afflicted with Candida infections. The fact that different bacterial strains exert a preventative effect on different vaginal disorders is surprising and well appreciated.

Women afflicted with bacterial vaginosis may thus administer an administration unit comprising L. casei, and especially LN 113 as its main bacterial component, whereas women afflicted by Candida infections should administer an administration unit comprising as its main bacterial constitutent L. fermentum, e.g. LN 99.

In embodiments, an administration unit according to the invention further comprises a strain selected from the group consisting of Lactobacillus gasseri, Lactobacillus crispatus and Pediococcus acidilactici.

Especially, the strain is selected from the group consisting of the strain of Lactobacillus gasseri, LN 40, deposited under number LMG P-20560, the strain of Lactobacillus crispatus, LN 01, deposited under number LMG P-20558, and the strain of Pediococcus acidilactici, LN 23, deposited under number LMG P-20559.

These strains, along with LN 99 and LN 113, have a high affinity towards the vaginal epithelium, and thus a high ability to colonize vagina.

An administration unit according to the present invention may comprise a mixture of different types of lactic acid bacterial strains.

The present inventors have found that the relationship between the strains LN 113, LN 99, LN 40, and LN 23 may be varied to selectively prevent and/or treat BV, and Candida infections, respectively.

If the administration unit should serve as a treatment and/or prophylaxis for Candida infections, the ratio of the strain Lactobacillus fermentum, LN 99, deposited under number LMG P-20561 to the strains of Lactobacillus gasseri, LN 40, deposited under number LMG P-20560, Lactobacillus casei subsp rhamnosus, LN 113, deposited under number LMG P-20562, and the strain of Pediococcus acidilactici, LN 23, deposited under number LMG P-20559 may be 50:30:10:10.

This ratio between the bacterial strains is particularly advantageous for the treatment of Candida infections.

In contrast, if the administration unit should serve as a treatment and/or prophylaxis for bacterial vaginosis, a suitable ratio of the strain Lactobacillus casei subsp rhamnosus, LN 113, deposited under number LMG P-20562 to said strains of Lactobacillus fermentum, LN 99, deposited under number LMG P-20561, Lactobacillus gasseri, LN 40, deposited under number LMG P-20560, and the strain of Pediococcus acidilactici, LN 23, deposited under number LMG P-20559 is 50:30:10:10

In embodiments, the administration unit according to the invention further comprises a second envelope arranged between the core and the first envelope. This second envelope comprises at least one lipid.

The arrangement of a second envelope comprising at least one lipid helps to preserve and protect the bacteria from oxygen and moisture when present in the atmosphere, and a better bacterial stability is thereby achieved.

In embodiments of the invention, the first envelope may further comprise at least one prebiotic, which selectively promotes the growth of lactic acid bacteria. Such prebiotics may be selected from a fructose-containing oligosaccharide, a galacto-oligosaccharide, synthetic lactulose, and amylopektin. These specific prebiotics are selectively promotive of lactic acid bacteria growth without promoting the growth of pathogens. The survival and reproduction of the LAB are thereby improved.

Upon degradation of the first envelope in the vagina, such prebiotics are released and help to create a favourable environment for the lactic acid bacteria when these are released.

In alternative embodiments, the first envelope further comprises a pH-buffering agent.

The pH-lowering effect extered by the biodegradable and biocompatible polymer upon degradation may be further enhanced by the presence of a pH-buffering agent in the first envelope.

In another aspect, the present invention relates to an administration unit having the above-mentioned characteristics
- for use as a medicinal product
- in the manufacture of a medicinal product for the prophylaxis and/or treatment of a disorder in the urogenital tract
- for the prophylaxis and/or treatment of a disorder in the urogenital tract,
   wherein the disorder in the urogenital tract is typically Candida vaginitis or bacterial vaginosis.

The invention also relates to a method for the the prophylaxis and/or treatment of a disorder in the urogenital tract, comprising administering to a patient in need thereof a pharmaceutically effective amount of an administration unit having the above mentioned characteristics.

In yet another aspect, the present invention relates to the use of Lactobacillus fermentum, LN 99, deposited under number LMG P-20561 in the manufacture of a medicinal product for the prophylaxis and/or treatment of Candida vaginitis, and to a method for the the prophylaxis and/or treatment of Candida vaginitis, comprising administering to a patient in need thereof a pharmaceutically effective amount of Lactobacillus fermentum, LN 99, deposited under number LMG P-20561.

Further, the invention relates to the use of Lactobacillus casei subsp rhamnosus, LN 113, deposited under number LMG P-20562, in the manufacture of a medicinal product for the prophylaxis and/or treatment of bacterial vaginosis, and to a method for the the prophylaxis and/or treatment of bacterial vaginosis, comprising administering to a patient in need thereof a pharmaceutically effective amount of Lactobacillus casei subsp rhamnosus, LN 113, deposited under number LMG P-20562.

These and other aspects of the invention will be apparent from and elucidated with reference to the embodiment(s) and examples described hereinafter.

### Brief description of the drawings

Fig 1 is a schematic view illustrating an administration unit according to the present invention.
Fig 2 is a comparison on the inhibition of Candida albicans by different lactic acid bacterial strains in vitro.
Fig 3 is a detailed outline of an in vivo study performed on 37 women with respect to long-term colonization of the strains LN 40, LN 99, LN 113, and LN 23.
Fig 4 is a graph showing the distribution of the four strains LN 40, LN 99, LN 113, and LN 23 in women afflicted with bacterial vaginosis.

### Detailed description

It is of great importance to maintain the balance between the microorganisms in the vagina. In the treatment of vaginal infections it is important to re-establish the LAB-dominated biota.

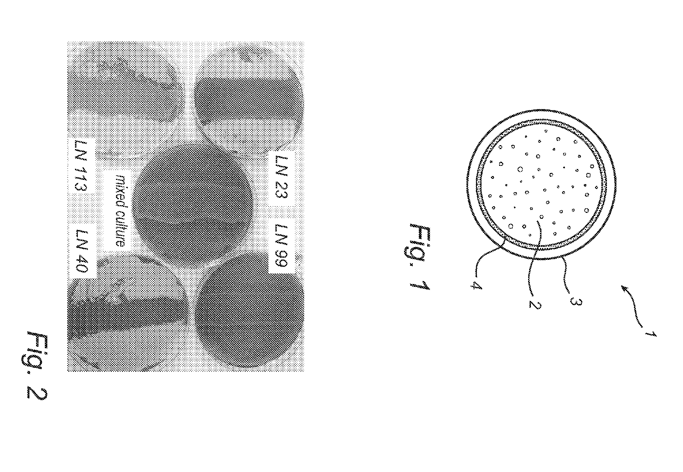

Referring to figure 1, the present invention relates to an administration unit 1 comprising a core comprising at least one lactic acid producing bacteria 2; said core being surrounded by a first envelope 3 comprising a biodegradable and biocompatible polymer.

As used herein, the term "administration unit" means a pill, tablet, capsule, lozenge, suppository or an ovule.

As used herein the term "lactic acid producing bacteria" or "LAB" means bacteria that by fermentation produce lactic acid.

As used herein the term "biodegradable and biocompatible polymer" refers to a polymer which gradually loses its structural integrity; i.e. degrades in the moist milieu of the vagina without eliciting any undesirable local or systemic effects in the subject of that therapy. Such a polymer should also be compatible with the lactic acid bacteria comprised within the administration unit.

An administration unit according to the invention is highly efficacious in killing undesired pathogens in the vagina and helps to maintain and re-establish a healthy and non-pathogenic bacterial flora in the vagina. The administration unit of the invention may be administered at any time during a woman's menstrual cycle in contrast to many prior art absorbing articles comprising LAB, which only are applied during menstruation. Hence, an improved prophylactic effect is achieved by an administration unit according to the invention.

The administration unit of the invention may exist in various shapes, but typically exist in an oval, spherical, ellipsoidal or circular form.

The diameter of the unit is typically from 5 mm to 25 mm.

The biodegradable and biocompatible polymer of the invention preferably exerts a pH buffering effect upon degradation in the vagina.

Typically, this pH buffering effect is achieved by lowering the pH in the vagina. A pH within the range from 3.8 to 5.0, e.g. 3.8 to 4.3 is preferred as the lactic acid bacteria require a moderately acid environment to operate.

The biodegradable and biocompatible polymer may be any polymer which degrades in the moist milieu of the vagina without eliciting any undesirable effects in the subject.

Preferably, polylactic acid is used.

The use of PLA has a number of advantages. First, upon exposure to moist (e.g. in the vagina), it degrades into lactic acid, which may act as a growth substrate for lactic acid bacteria and which lowers the pH; i.e. creates a slightly acidic environment in the vagina. These conditions are ideal for the lactic acid bacteria to exert their protective and inhibitory action against undesired pathogens. Second, PLA adheres well to epithelial cells in the vagina, which is an important property for the effect of the invention. The ability of PLA to enhance cell adhesion is discussed in Watanabe et al; "Cell engineering biointerface focusing on cytocompatibility using phospolipid polymer with an isomeric oligo (lactic acid) segment", Biomacromolecules, Vol 6, No 3, pp 1797-1802, 2005.

The family of poly lactic acids includes the pure poly-L- lactic acid, the pure poly-D-lactic acid and the poly-D, L lactic acid (DL-PLA). The hydrolysis of PLA to lactic acid is catalyzed by enzymes produced by bacteria in the vagina and/or vaginal epithelial cells.

In embodiments of the invention, polylactic acid is typically admixed or crosslinked with other components, which e.g. may affect the degree of degradation. For example, polylactic acid may be organized into diblocks or triblocks with ethylene glycol and/or glycolic acid comonomers or triblocks.

As used herein the term "comprises a biodegradable and biocompatible polymer" means that the first envelope may contain, in addition to other optional components, such a polymer. It also means that the first envelope may comprise a copolymer which comprises a biodegradable and biocompatible polymer, e.g. PLA. For example, a copolymer comprising PLA crosslinked to other polymer(s) and components are included within this definition.

The rate of degradation; i.e. the rate of hydrolysis of the first envelope may thus be varied by the selective choice of such components.

Other suitable biodegradable and biocompatible polymers include alginates, such as sodium alginate, hyaluronic acid, poly(ethylene glycol) (PEG), kappa-carrageenan, collagen and dextrans.

The administration unit according to the invention may further comprise conventional consistency agents or pharmaceutical excipients, such as hydroxyl propyl methyl cellulose, carboxy methyl cellulose, microcrystalline cellulose, gelatin, silicon dioxide, magnesium stearate and various types of gums, which provide for a suitable consistency of the administration unit. Any suitable consistency agent or pharmaceutical excipient may be added which is biocompatible and does not result in any undue toxicity.

An administration unit according to the invention is solid or semi/solid at room temperature and in a dry environment, but degrades at body temperature and in contact with body fluids.

Typically, the administration unit has a relatively porous and "sponge-like" consistency. This allows for a larger surface of exposure to moist, which thereby enhances the penetration of water and rate of hydrolysis.

It is desired that the administration unit gradually melts in the vagina; i.e. that there is a delayed release of the lactic acid bacteria. By varying the properties of the first envelope; i.e. the amount of PLA, the admixture or crosslinking with other componens, as previously mentioned, and the thickness of the first envelope, the release rate of the bacteria may be adjusted.

Advantageously, the lactic acid bacteria are released 90 min to 48 h, e.g. 90 min to 24 h after administration.

Typically, at least 90 minutes are required for the bacteria to mature and adapt themselves to growth conditions (bacterial lag phase). In contrast, vaginal epithelial cells are typically replaced after 48 hours, and adherance of the administration unit to the vaginal epithelium is no longer significant.

The thickness of the first envelope may be in the range of 5 µm to 5 mm.

The administration unit may be administered orally or vaginally. The vaginal route of administration is preferred since a local preventative and protective effect is desired.

Before administration, the administration unit may be dipped in water to render the unit somewhat "sticky" and/or soft for more comfortable handling and insertion. A better adhesion to the vaginal cell walls may thus be achieved and, if PLA is used, the hydrolysis into lactic acid is slightly initiated before administration.

The concentration of the lyophilised LAB is typically at least 10⁴ cfu/unit; e.g. at least 10⁶ cfu/unit. The LAB concentration may be varied depending on the desired effect and the severity of the condition. For example, in some instances it may be desired to administer a "boost" dose of an administration unit according to the invention. In this case, the concentration of the LAB is higher, e.g. at least 10⁸ cfu/unit.

Alternatively, a prophylactic effect is desired and a lower concentration of LAB may be chosen. Hence, the dose regimen of the administration unit of the invention may vary from 1 to 15 administration units per treatment period.

Preferably, the bacteria originate from the urogenital tract of a woman with a normal flora of microorganisms.

The lactic acid producing bacteria may be selected from the genus Lactobacillus, Pediococcus, Lactococcus and Leuconostoc, which all have a beneficial and protective mode of action against undesired pathogens in the vagina.

In one embodiment of the invention, the lactic acid bacteria is Lactobacillus fermentum, e.g. the strain Lactobacillus fermentum, LN 99, deposited by the Applicant under number LMG P-20561.

The present inventors have found that Lactobacillus fermentum, especially the strain LN 99 is particularly effective for the treatment and/or prophylaxis of Candida vaginitis.

Inhibition tests in vitro have shown that, compared to other lactic acid bacterial strains, LN 99 is superior in the inhibition of Candida albicans (see Example 1).

The finding that L. fermentum, preferably LN 99, is selective for the yeast Candida albicans is surprising, and an administration unit comprising Lactobacillus fermentum, particularly the strain LN 99, may be administered to women afflicted with Candida infections for the treatment and/or prophylaxis thereof.

Another important finding by the present inventors is that a strain of Lactobacillus casei, especially the strain Lactobacillus casei subsp rhamnosus, LN 113, deposited by the applicant under number LMG P-20562, is particularly suitable for the treatment and/or prophylaxis of bacterial vaginosis.

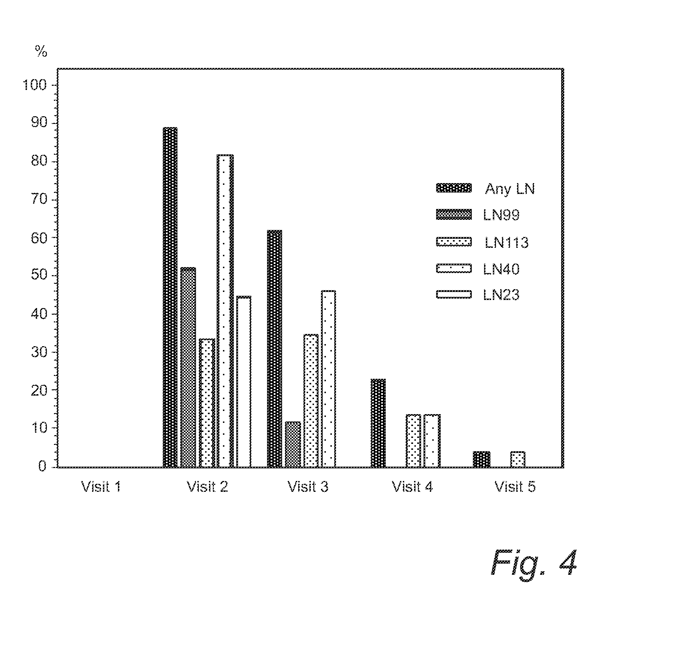

Comparative tests have been performed (see Example 2), which show that LN 113 produces a large amount of L-lactic acid, which is believed to be the most biologically important lactic acid isomer and an indicator of the protective mechanisms exerted by LAB in the vaginal milieu. Furthermore, LN 113 inhibits several vaginal pathogens and has an increased tendency to colonize in women afflicted with bacterial vaginosis (Fig. 4).

Hence, an administration unit comprising L. casei, e.g. LN 113 may be administered to women afflicted by bacterial vaginosis for the treatment and/or prophylaxis thereof.

The present invention is not limited to the use of L. fermentum or L. casei, respectively, as the sole bacterial component. In contrast, a mixture of several types of LAB may be used. An administration unit according to the invention may e.g. further comprise a strain selected from the group consisting of Lactobacillus gasseri, Lactobacillus crispatus and Pediococcus acidilactici.

The following strains, deposited by the Applicant, are preferably selected:
- Lactobacillus gasseri, LN 40, deposited under number LMG P-20560
- Lactobacillus crispatus, LN 01, deposited under number LMG P-20558, and
- Pediococcus acidilactici, LN 23, deposited under number LMG P-20559.

These strains, along with the strains LN 113 and LN 99, previously discussed have the ability to to colonize and become established in a human vagina, even during menstrual discharge.

The present inventors have made a comprehensive analysis on the long-term colonization ability of these strains on women diagnosed with Candida vaginitis or bacterial vaginosis, and found that these strains have a particularly high affinity towards the vaginal epithelium and remarkable colonization ability (see Example 3).

As previously mentioned, women afflicted with bacterial vaginosis are often treated the same way as wome afflicted with Candida infections.

Although all of the strains LN 113, LN 99, LN 40, LN 01, and LN 23 are effective against disorders in the urogenital tract by exerting an inhibitory action against undesired pathogens, the present inventors have found that the relationship between these strains may be varied in order to selectively inhibit Candida albicans, and bacteria associated with bacterial vaginosis, respectively.

Women afflicted with Candida infections should thus administer an administration unit comprising LN 99 as its main bacterial component, whereas women afflicted by BV should administer an administration unit comprising, as its main bacterial constitutent, LN 113.

A suitable ratio of LN 99 to the strains LN 40, LN 113, and LN 23 for the treatment of Candida infections is 50:30:10:10.

A suitable ratio of LN 113 to the strains LN 99, LN 40, and LN 23 for the treatment of BV is 50:30:10:10.

An administration unit according to the invention may further comprise a second envelope arranged between the core and the first envelope. This second envelope, denoted 4 in figure 1, comprises at least one lipid.

The lipid acts to preserve and protect the bacteria from oxygen and moisture in the atmosphere, and a better bacterial stability is thereby achieved. The present invention is not limited to a specific type of lipid, but any type of fat, wax or oil may be used as long as it does not confer any undesired or toxic side effects.

The first envelope of the administration unit according to the invention may also comprise at least one prebiotic, which selectively promotes the growth of lactic acid bacteria. Such prebiotics may be selected from a fructose-containing oligosaccharide, a galacto-oligosaccharide, synthetic lactulose, and amylopektin.

As used herein, the term "prebiotic" refers to a substance which is fermented by lactic acid bacteria and helps to increase their survival and reproduction. Prebiotics according to the invention selectively promote the growth of lactic acid bacteria without promoting the growth of pathogens.

Upon degradation of the first envelope in the vagina, such prebiotics are released and help to create a favourable environment for the lactic acid bacteria when these are released.

Amylopektin is preferably used since the osmotic pressure of amylopektin is similar to the osmotic pressure of the vaginal epithelial cells. Hence, the vaginal mucosa is not dried out upon prolonged exposure of the administration unit.

Prebiotics may also be admixed with the lactic acid bacteria of the core of the administration unit.

The first envelope may further comprise a pH-buffering agent to further enhance the pH-buffering effect exterted by the biodegradable and biocompatible polymer.

Examples of such pH-buffering agents include e.g. acetic acid, boric acid, citric acid, lactic acid, ascrobic acid and salts thereof.

Additional substances, e.g. carbohydrates such as maltodextrin, glycogen, starch, glucose, fructose, lactose, dextrose, maltose, lactulose, mannose and vitamins may also be included in the administration unit of the invention.

Maltodextrin is advantageously used since it may act as a growth factor for LAB and also has an its anti-emulsive effect.

The administration unit according to the present invention reinforces the natural protection mechanisms of the vaginal mucosa by establishing a productive environment for lactic acid bacteria, lowering the pH and also by exerting an inhibiting action on a number of undesired microbes, e.g. C. albicans, E. coli, S. aureus, G. vaginalis, and Prevotella species.

Furthermore, an administration unit according to the invention is easy, convenient and comfortable to administer.

Additional substances, such as adjuvants, carriers, preservatives, vitamins, minerals, oestrogen etc may also be added and these substances are well known to persons skilled in the art.

In embodiments, the present invention relates to an administration unit for use as a medicinal product.

Further, an adminstration unit having the above mentioned characteristics may be used in the manufacture of a medicinal product for the prophylaxis and/or treatment of a disorder in the urogenital tract.

By a "disorder in the urogenital tract" is meant any disturbance or disorder in the urinary tract or in the genital organs in a female. In particular, the invention is suited for the treatment of conditions in the vagina.

By "prophylaxis and/or treatment of a disorder" is meant any treatment in order to cure or alleviate a disorder according to the above, or to prevent the development of such a disorder.

The disorders typically include Candida vaginitis and bacterial vaginosis.

However, examples of other urogenital disorders include urinary tract infections, intermediate vaginal microbiota, chlamydia, genital mycoplasma, trichomoniasis, pelvic inflammatory disease, HIV, gonococci and infections caused by human papillomavirus (HPV).

In embodiments, the present invention relates to an administration unit for the prophylaxis and/or treatment of a disorder in the urogenital tract, e.g. Candida vaginitis and BV.

The invention also relates to a method for the the prophylaxis and/or treatment of a disorder in the urogenital tract, comprising administering to a patient in need thereof a pharmaceutically effective amount of an administration unit according to the above.

By a "pharmaceutically effective amount" is meant an amount of the administration unit according to the invention, which will lead to the desired pharmacological and/or therapeutic effect. The desired pharmacological and/or therapeutic effect is, as stated above, to cure or alleviate and/or prevent the development of a disorder in the urogenital tract.

By a "patient" is meant any human or non-human mammal, female or male, in need of being treated with the administration unit and/or method according to the invention.

In embodiments, the present invention also relates to the use of Lactobacillus fermentum, LN 99, deposited under number LMG P-20561 in the manufacture of a medicinal product for the prophylaxis and/or treatment of Candida vaginitis.

The invention also relates to a method for the prophylaxis and/or treatment of Candida comprising administering to a patient in need thereof a pharmaceutically effective amount of Lactobacillus fermentum, LN 99, deposited under number LMG P-20561.

In alternative embodiments, the present invention relates to the use of Lactobacillus casei subsp rhamnosus, LN 113, deposited under number LMG P-20562, in the manufacture of a medicinal product for the prophylaxis and/or treatment of bacterial vaginosis and to a method for the prophylaxis and/or treatment of bacterial vaginosis comprising administering to a patient in need thereof a pharmaceutically effective amount of Lactobacillus casei subsp rhamnosus, LN 113, deposited under number LMG P-20562.

### Examples

### Example 1: Inhibition of Candida albicans in vitro

### Inhibitory activity of LN lactic acid bacteria strains against Candida albicans on solid culture medium

The antagonistic properties of freeze dried LN23, LN40, LN99 and LN113 against Candida albicans were preliminary tested using a modified agar overlay method (Fitzsimmons, "Inhibition of Candida albicans by Lactobacillus acidophilus: evidence for the involvement of a peroxidase system, Microbios. 1994;80(323):125-33; Strus et al, The in vitro activity of vaginal Lactobacillus with probiotic properties against Candida, Infect Dis Obstet Gynecol. 2005 June; 13(2): 69-75).

The yeast was isolated from a woman with Candida vaginitis. 50µl of MRS broth overnight culture of the LN strains were cultured as an approximately 2 cm wide stripe on 15 ml MRS agar. The plates were incubated at 37°C for 48h at anaerobic conditions. The cultures were then overlaid with 10ml Malt agar. Overnight culture of C. albicans (in Sabouraud broth) was diluted with sterile PBS, pH 7.4, to approximately 0.5 McFarland's score. 200 µl of the diluted yeast culture was then streaked over the Malt agar layer. After incubation anaerobicaly for 24h at 37°C, the plates were left at room temperature for 48h. The result was determined in form of inhibition zones: complete inhibition of C. albicans over the LN strain and beyond (++), inhibition of C. albicans over the LN strain (+), and lack of inhibition (-).

### Results

The ability of the LN strains to inhibit growth of Candida albicans on a Malt agar overlay on MRS agar were different (figure 2). All the LN strains were able to inhibit the yeast growth, but while the inhibition by LN 23, LN 40 and LN 113 were detected only over the lactic acid bacteria stripe, the inhibition by LN 99 and by the mixed culture was detected not only over the LN cultures but even beyond the cultures, over the whole plates.

### Inhibitory activity of culture supernatants of LN lactic acid bacteria strains against Candida albicans

The inhibitory activity of the different LN strains against C. albicans was also tested in culture supernatants. 40ml MRS-broth was inoculated with 400µl (1 % V/V) overnight culture of LN23, LN40, LN99, LN113 and with a mix of these cultures (100 µl of each cultures), and incubated at 37°C for 24h. The cultures were then centrifuged at 5000 rpm for 30min. The pH of the supernatants was measured by pH-meter. 10 ml of the supernatants were then filtered through 0.2µm sterile filters. The pH of the remainder supernatants were then adjusted to the initial pH value of the MRS-broth (pH 6.2) using 2M NaOH. 10 ml of these supernatants were then also filtered through 0.2 µm sterile filters. The pH level of 10 ml pure MRS-broth was adjusted to acidic pH (pH 4.1, same pH level as the medium pH level of the culture supernatants) using 1 M HCl, and then sterile filtered.

10ml pure MRS (pH 6,2) and the acidified MRS-broth (4.1), as well as the sterile filtered supernatants (both with acidic pH and pH 6.2) were then inoculated with 100µl overnight culture of C. albicans (approximately 0.5 McFarland's score), and incubated for 24 h at 37°C. The optic density of the cultures was measured at wavelength 550 nm using spectrophotometer. The CFU was quantified by spreading 100µl of the cultures to MRS agar plates. The inhibition was evaluated visually, and by measuring the optical density of the cultures at wavelength 550 nm using spectrophotometer, and by culturing 100µl of the yeast cultures on MRS agar plates, and counting the colonies.

### Results

When the pH of the bacterial culture supernatants was acidic (pH 3.9; 4.0; 4.3; 4.3 and 4.2), Candida albicans was not growing, not even in MRS-broth with acidic pH (pH adjusted to 4.1).

When C. albicans was cultured in the sterile filtered, pH adjusted (pH 6.1) supernatants, it was not growing in the supernatant of LN 99 and of the mixed culture, but was well growing in the supernatants of the other LN strains (LN23, LN40 and LN113), showing only a slightly inhibition of the yeast, compared with the growth in MRS-broth. The inhibition was clearly visible, but even detected by comparing the OD₅₅₀ and CFU ml⁻¹ of the yeast cultures (Table 1).

**Table 1: Inhibition of Candida albicans in sterile acidic, and in pH adjusted supernatants by different LN lactic acid bacteria**

| | *C*. *albicans* cultured in the acidic, sterile filtered supernatant of the bacteria cultures | | *C*. *albicans* cultured in sterile filtered, pH adjusted (pH 6.2) supernatant of the bacteria cultures | | |
|---|---|---|---|---|---|
| Lactic acid bacteria strains cultured in MRS broth | Growth of *C*. *albicans* | | Growth of *C*. *albicans* | OD₅₅₀ | CFU ml⁻¹ |
| LN23 | - | | + | 0,640 | 3,0 · 10⁶ |
| **LN99** | - | | - | **0,122** | **3,7 · 10⁵** |
| LN113 | - | | + | 0,792 | 4,1 · 10⁶ |
| LN40 | - | | + | 0,690 | 3,8 · 10⁶ |
| **Mixed culture** | - | | - | **0,128** | **5,4 · 10⁵** |
| | | | | | |

| | *C*. *albicans* cultured in acidified MRS broth | | *C*. *albicans* cultured in pure MRS broth | | |
|---|---|---|---|---|---|
| X | Growth of *C*. *albicans* | | Growth of *C*. *albicans,* | OD₅₅₀ | CFU ml⁻¹ |
| X | - | | + | 0,789 | 4,3 · 10⁶ |

### Discussion

The in vitro studies of the lactic acid bacteria strains (LN 23, LN 40, LN 99, LN 113) showed that the strains and the mix of the strains have the ability to inhibit the growth of Candida albicans both on solid and in liquid MRS culture medium. All the LN strains and the mix of the strains lowered the pH of the liquid culture media with approximately 2.0 pH value. The acidic pH is the result of the acid production in the metabolic pathway.

The inhibitory properties of LN 99 differ from the other LN strains, inhibiting the yeast not only over the bacteria stripes, but even beyond, when it was cultured on MRS agar plates (see Fig 2).

Culturing Candida in sterile filtered supernatant of the LN strains, a clear inhibition of the yeast was observed. The growth of C. albicans was strongly inhibited in pH adjusted (pH 6.2), sterile filtered supernatant of LN 99, whereas the supernatants of the other strains, showed only a slightly inhibition against the yeast cultured at the same conditions. The mixed culture of the LN strains showed the same antagonistic properties as the LN 99. Accordingly, LN 99 could positively influence a disturbed vagina with Candida overgrowth

### Example 2: Inhibitory activity of LN lactic acid bacteria strains against pathogens and production of L- and D lactic acid

### L- and D lactic acid production by the LN strains

40 ml MRS-broth (initial pH value 6.1) was inoculated with 400 µl (1 % v/v) cultures of freeze dried LN 23, LN 40, LN 99, LN 113 and with a mix of these strains (100 µl of each cultures), then incubated for 24 h at 37 °C. 1ml samples of the supernatants were used for 10 fold dilutions that were spread to MRS agar plates for culturing and quantification of CFU mr⁻¹ culture. The remaining cultures were then centrifuged at 5000 rpm for 30 min. The production of lactic acid by the different strains was evaluated by measuring the pH of the supernatant, and by the quantification of produced D-and L-lactic acid. The amount of D-and L-lactic acid were analysed by using the D-/L-Lactic Acid (D-/L-Lactate) enzyme assay kit (Megazyme).

### Inhibitory activity against bacterial species

The inhibitory activity of LN 23, LN 40, LN 99 and LN 113 was tested against *Escherichia coli, Streptococcus agalactiae, Enterococcus faecalis, Staphylococcus aureus,* and against each other LN strains. The inhibitory activity was detected by spot-inoculating the bacteria LN 23, LN 40, LN 99, LN 113 and a mix of these strains onto MRS agar plates. The plates were then incubated anaerobically at 37°C for 24 h. 10 ml MRS soft agar (0,75% agar) was inoculated with 100 µl approximately 10⁷ cells ml⁻¹ of overnight cultures of indicator organism. The MRS agar plates with developed colonies of producer strains were then overlaid with the soft agar containing the indicator strains, and the plates were incubated anaerobically at 37°C for 48 h. The indicator lawns were then detect for zones of inhibition. A clear zone of inhibition of least 0.2 mm in diameter was recorded as positive.

### Results

Acidification in form of decreased pH was detected in all of the culture supernatants. The final pH of culture supernatants was 3.9, 4.0, 4.3 and 4.3 in order to LN 23, LN 40, LN 99 and LN 113. The final pH of the supernatant of the mixed cultures was 4.2. All LN strains produced D-and L-lactic acids in very similar amounts, apart from LN113, which produced D-lactic acid only in very small amounts, but L-lactic acid in large amounts. The highest amount of lactic acid, mg CFU⁻¹, was produced by LN113 (Table 2).

**Table 2: Production of lactic acid**

| | LN 23 | LN 99 | LN 113 | LN 40 | Mixed culture |
|---|---|---|---|---|---|
| CFU ml⁻¹ culture | 6x10⁹ | 3x10⁹ | 10⁹ | 2x10⁹ | 6x10⁹ |
| pH supernatant | 3.9 | 4.3 | 4.3 | 4.0 | 4.2 |
| D-lactic acid (mg ml⁻¹) | 6.00 | 4.82 | 0.25 | 7.03 | 5.24 |
| L-lactic acid (mg ml⁻¹) | 9.61 | 4.48 | 7.82 | 5.77 | 5.51 |
| Total lactic acid (mg ml⁻¹) | 15.61 | 9.30 | 8.07 | 12.80 | 10.75 |
| D-lactic acid (mg CFU⁻¹) | 1.00x10⁻⁹ | 1.61x10⁻⁹ | 2.50x10⁻¹⁰ | 3.52x10⁻⁹ | 8.73x10⁻¹⁰ |
| L-lactic acid (mg CFU⁻¹) | 1.60x10⁻⁹ | 1.50x10⁻⁹ | 7.82x10⁻⁹ | 2.88x10⁻⁹ | 9.18x10⁻¹⁰ |
| Total lactic acid (mg CFU⁻¹) | 2.60x10⁻⁹ | 3.11x10⁻⁹ | 8.07x10⁻⁹ | 6.40x10⁻⁹ | 1.79x10⁻⁹ |

LN 23, LN99, LN 113 and a mix of the all LN strains showed approximately the same inhibitory activity against *Streptococcus agalactiae, Enterococcus faecalis* and *Staphylococcus aureus* (Table 3).

**Table 3: Summary of results**

| | LN 23 | LN 99 | LN 113 | LN 40 | Mixed culture |
|---|---|---|---|---|---|
| lactic acid production | + | + | + | + | + |
| D - lactic acid | + | + | +/- | + | + |
| L - lactic acid | + | + | ++ | + | + |
| inhibition of pathogens* | + | + | + | - | + |
| inhibition of the other LN sp | - | - | - | - | X |

### Discussion

The in vitro studies showed that all the LN strains (LN 23, LN 40, LN 99, LN 113) produced lactic acid and lowered the pH of the culturing media with approximately 2.0 value of pH. The strains produced both D-and L-lactic acid, apart from LN 113, which produced almost exclusively L-lactic acid, which is believed to be the most biologically important isomer and a good indicator of the protective mechanisms exerted by LAB in the vaginal milieu.

Furthermore, LN 23, LN 99 and LN 113 have good inhibitory effect against the pathogens tested in this study. The strains do not have any inhibitory effect against other LN strains.

### Example 3: Long-term colonization of the strains LN 40, LN 99, LN 113, LN 01, and LN 23 in vivo

37 women diagnosed with Candida vaginitis or Bacterial vaginosis were included in the study. Following local clotrimazole or clindamycine treatment for three consecutive days, these women were administered a combination of the strains L. fermentum, denoted by the applicant as LN99, L. casei ssp rhamnosus, denoted by the applicant as LN113, L. gasseri, denoted by the applicant as LN40, and P. acidilactici, denoted by the applicant as LN23. The bacteria were administered in the form of capsules. The capsules were applied in the vagina during 5 days.

21 patients received capsules with the above bacterial strains and 16 received placebo capsules.

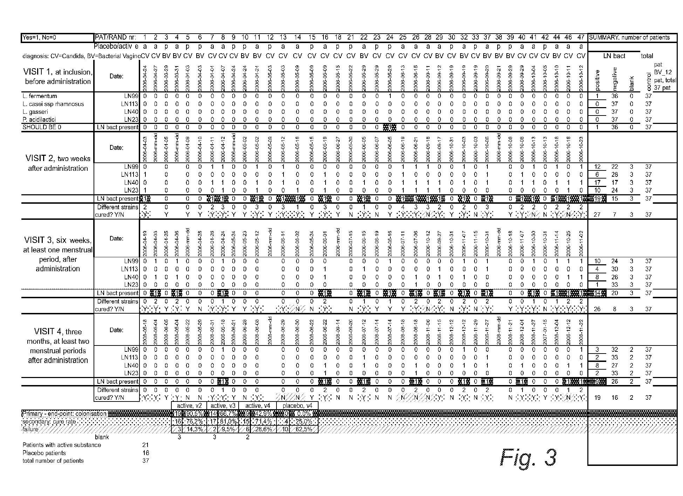

The presence of each of the inventive strains was evaluated (i) before treatment, after (ii) 2 weeks, (iii) 6 weeks, i.e. one menstrual period after administration, and (iv) 3 months, i.e. at least 2 menstrual periods after administration using molecular techniques which identifies the bacteria down to the specific strain level. See figure 3 for an overview of the study.

Two weeks after administration, 19 of 21 patients (90,5%) had at least one of the strains present in the vagina.

After one menstrual period, i.e. 6 weeks after administration, 14 of 21 patients (66,7%) had at least one of the strains present in the vagina.

Three months after administration, a time period encompassing at least two menstrual periods, 9 of 21 patients (42,9%) had at least one of the strains present in the vagina.

These results are remarkable since, to our knowledge, no previous studies have explicitly shown the ability of the bacteria to colonize over such a long time period, or shown that the lactic acid producing bacterial strains are able to establish in the vagina even during menstrual discharge.

It is also worth mentioning that the cure rate during these three separate occasions was: 76,2 % (after 2 weeks), 81,0 % (after 6 weeks), and 71,4 % (after 3 months). The cure rate of the placebo treated women was 25% after 3 months.

## Claims

1. An administration unit comprising a core comprising at least one lactic acid producing bacteria; said core being surrounded by a first envelope comprising a biodegradable and biocompatible polymer.

2. An administration unit according to claim 1, wherein said biodegradable and biocompatible polymer exerts a pH buffering effect upon degradation in the vagina.

3. An administration unit according to claim 1 or 2, wherein said biodegradable and biocompatible polymer is polylactic acid.

4. An administration unit according to any one of the preceding claims, wherein said administration unit is administered vaginally.

5. An administration unit according to any one of the preceding claims, wherein said lactic acid producing bacteria are freeze-dried.

6. An administration unit according to any one of the preceding claims, wherein said lactic acid producing bacteria are selected from the genus Lactobacillus, Pediococcus, Lactococcus and Leuconostoc.

7. An administration unit according to any one of the preceding claims, wherein said lactic acid producing bacteria is a strain of Lactobacillus fermentum.

8. An administration unit according to claim 7, wherein said strain of Lactobacillus fermentum is the strain Lactobacillus fermentum, LN 99, deposited under number LMG P-20561.

9. An administration unit according to any one of the preceding claims, wherein said lactic acid producing bacteria is a strain of Lactobacillus casei.

10. An administration unit according to claim 9, wherein said strain of Lactobacillus casei is the strain Lactobacillus casei subsp rhamnosus, LN 113, deposited under number LMG P-20562.

11. An administration unit according to any of the preceding claims, further comprising a strain selected from the group consisting of Lactobacillus gasseri, Lactobacillus crispatus and Pediococcus acidilactici.

12. An administration unit according to claim 11, wherein said strain(s) is (are) selected from the group consisting of the strain of Lactobacillus gasseri, LN 40, deposited under number LMG P-20560, the strain of Lactobacillus crispatus, LN 01, deposited under number LMG P-20558, and the strain of Pediococcus acidilactici, LN 23, deposited under number LMG P-20559.

13. An administration unit according to claim 12, wherein the ratio of said strain of Lactobacillus fermentum, LN 99, deposited under number LMG P-20561 to said strains of Lactobacillus gasseri, LN 40, deposited under number LMG P-20560, Lactobacillus casei subsp rhamnosus, LN 113, deposited under number LMG P-20562, and the strain of Pediococcus acidilactici, LN 23, deposited under number LMG P-20559 is 50:30:10:10.

14. An administration unit according to claim 12, wherein the ratio of said strain of Lactobacillus casei subsp rhamnosus, LN 113, deposited under number LMG P-20562 to said strains of Lactobacillus fermentum, LN 99, deposited under number LMG P-20561, Lactobacillus gasseri, LN 40, deposited under number LMG P-20560, and the strain of Pediococcus acidilactici, LN 23, deposited under number LMG P-20559 is 50:30:10:10.

15. An administration unit according to any one of the preceding claims further comprising a second envelope arranged between said core and said first envelope; said second envelope comprising at least one lipid.

16. An administration unit according to any one of the preceding claims, wherein said first envelope further comprises at least one prebiotic, which selectively promotes the growth of lactic acid bacteria; said prebiotic is selected from a fructose-containing oligosaccharide, a galacto-oligosaccharide, synthetic lactulose, and amylopektin.

17. An administration unit according to any one of the preceding claims, wherein said envelope further comprises a pH-buffering agent.

18. An administration unit according to any one of the claims 1-17 for use as a medicinal product.

19. Use of an administration unit according to any one of the claims 1-17 in the manufacture of a medicinal product for the prophylaxis and/or treatment of a disorder in the urogenital tract.

20. Use according to claim 19, wherein said disorder is Candida vaginitis.

21. Use according to claim 19, wherein said disorder is bacterial vaginosis.

22. An administration unit according to any one of the claims 1-17 for the prophylaxis and/or treatment of a disorder in the urogenital tract.

23. An administration unit according to claim 22, wherein said disorder is Candida vaginitis.

24. An administration unit according to claim 22, wherein said disorder is bacterial vaginosis.

25. A method for the the prophylaxis and/or treatment of a disorder in the urogenital tract, comprising administering to a patient in need thereof a pharmaceutically effective amount of an administration unit according to any one of the claims 1-17.

26. Use of Lactobacillus fermentum, LN 99, deposited under number LMG P-20561 in the manufacture of a medicinal product for the prophylaxis and/or treatment of Candida vaginitis.

27. Lactobacillus fermentum, LN 99, deposited under number LMG P-20561 for the prophylaxis and/or treatment of Candida vaginitis.

28. A method for the prophylaxis and/or treatment of Candida vaginitis comprising administering to a patient in need thereof a pharmaceutically effective amount of Lactobacillus fermentum, LN 99, deposited under number LMG P-20561.

29. Use of Lactobacillus casei subsp rhamnosus, LN 113, deposited under number LMG P-20562 in the manufacture of a medicinal product for the prophylaxis and/or treatment of bacterial vaginosis.

30. Lactobacillus casei subsp rhamnosus, LN 113, deposited under number LMG P-20562 for the prophylaxis and/or treatment of bacterial vaginosis.

31. A method for the prophylaxis and/or treatment of bacterial vaginosis comprising administering to a patient in need thereof a pharmaceutically effective amount of Lactobacillus casei subsp rhamnosus, LN 113, deposited under number LMG P-20562.
